# EUROPEAN PATENT APPLICATION

(11) **EP 3 925 636 A1**
(43) Date of publication of application: **22.12.2021**
(21) Application number: 20020280.2
(22) Date of filing: 17.06.2020
(51) Int. Cl.: A61L 15/44, A61L 15/42, A61L 15/60

(54) **COMPOSITE MEMBRANES CONTAINING A SMART-RELEASED CYTOPROTECTANT TARGETING THE INFLAMED TISSUE AND USE THEREOF**

(71) Applicant: Centre Of Experimental Medicine Slovak Academy Of Sciences Institute Of Experimental Pharmacology, 84104 Bratislava (SK)
(72) Inventor: Soltes, Ladislav, 82101 Bratislava (SK); Valachova, Katarina, 01841 Dubnica nad Vahom (SK); Mach, Mojmir, 85101 Bratislava (SK); Juranek, Ivo, 85101 Bratislava (SK)
(74) Representative: Jirotkova, Ivana

(57) **Abstract**

The invention describes self-associating biopolymers containing high-molar-mass hyaluronan and chitosan, which together form a carrier for a cytoprotectant L-(+)-ergothioneine, which is a biologically effective agent incorporated into this carrier. The final composite membranes containing L-(+)-ergothioneine are used for treatment of chronic skin lesions and difficult-to-heal wounds.

## Description

### Technical Field

The invention deals with a smart-released cytoprotectant - L-(+)-ergothioneine - from composite membranes consisting of self-associating biopolymers, whereas L-(+)-ergothioneine serves for treatment of chronic inflammatory skin lesions and difficult-to-heal skin wounds.

### Current state of the art

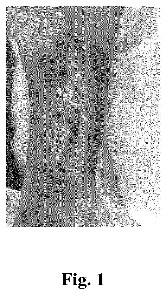

Chronic inflammatory skin damages - such as leg venous ulcers - [L. Collins, S. Seraj, Diagnosis and treatment of venous ulcers. American Family Physician 81 (2010) 989-996; https://www.bmj.com/content/328/7452/1358] are severe diseases afflicting patients longterm (Figure 1), which in case of diabetic patients can have fatal consequences - amputation of the limb.

Skin is a complex tissue, which is deficient in oxygen uptake at a site of inflammation. Lack of oxygen (state of hypoxia) is generally accompanied by formation of reactive oxygen species (ROS) such as superoxide anion radical O₂^{•-}, hydrogen peroxide H₂O₂, hydroxyl radical ^{•}OH, *etc.* These ROS may contribute to tissue damage so significantly that the process of healing is much longer and may result in collapse of the organism or death of a patient.

Inflamed skin, besides necrotic cells, contains cells deficient in oxygen uptake due to the damage of the cell extracellular glycocalyx. It is well known, that during hypoxia mitochondria in cells produce a great flux of ROS. Taking into account above mentioned facts, it appears to be appropriate to suppress formation of ROS at the site of chronic inflammation. Since the formation of ROS during hypoxia in tissues is substantially higher than in normoxia, restriction of oxygen uptake to the site of damage is counterproductive.

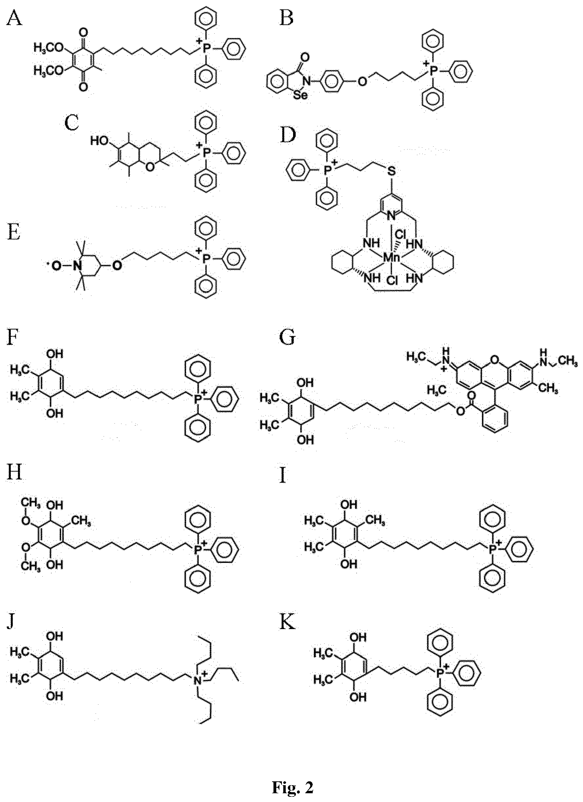

One way how to effectively reduce undesirable flow of ROS from mitochondria represents an application of the so-called mitochondrially-targeted antioxidants - MTAs. Several MTAs, which bear a positive ion of "Skulatchev type" (Figure 2; cations - usually P⁺ or N⁺), are already commercially available. Some mitochondrially targeted antioxidants are as follows: A - MitoQ, B - MitoPeroxidase, C - MitoE₂, D - MitoSOD, E - MitoTEMPOL, F - SkQ1H₂, G - SkQR1H₂, H - MitoQH₂, I - SkQ3H₂, J - SkQ4MH₂, K - SkQ5H₂ [adapted from J. Gruber, S. Fong, C.-B. Chen, S. Yoong, G. Pastorin, S. Schaffer, I. Cheah, B. Halliwell, Mitochondria-targeted antioxidants and metabolic modulators as pharmacological interventions to slow ageing. Biotechnology Advances 31 (2013) 563-592].

In the currently existing patents, one- or multi-component biocompatible polymeric membranes prepared to cover and treat wounds are mentioned. For example, the patent document JP 2004041586 describes material composed of chitosan and hyaluronic acid, which is highly biocompatible, elastic, adherent, and is used as a protective material for wounds.

The patent document CN 10408741 describes antiseptic adhesive bandage based on chitosan, which may involve hyaluronic acid as a polymer with better properties.

The patent DE 10135676 describes physiologically compatible permeable film, which contains predominantly cellulose as well as other components such as chitosan and hyaluronic acid. This film is used as a wound dressing, a "physiological" membrane, an artificial blood stream or a material for production of infusion tubes.

The patent document WO 2013/071235 describes biocompatible polymeric composition containing polyanionic polymers such as alginates or hyaluronates and polycationic polymers such as chitosan or dextran.

The patent document CN 102600495 describes composition comprising hyaluronic acid, collagen and chitosan with hemostatic effect on various wounds.

The patent document CN 102210885 describes material used as a wound dressing, which contains chitosan lactate and sodium hyaluronate.

Another patent document CN 103690989 describes hemostatic preparation as a wound dressing composed of chitosan, sodium hyaluronate and an antibacterial agent.

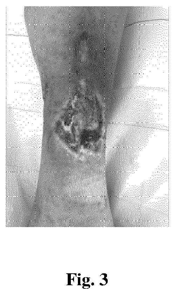

However, none of these documents solves the problem of difficult-to-heal wounds and chronic skin injuries caused primarily by reactive oxygen species (ROS). As depicted in Figure 3, application of an MTA, namely MitoQ, resulted in rapid healing of the wound in the patient (compare Figure 3 with Figure 1).

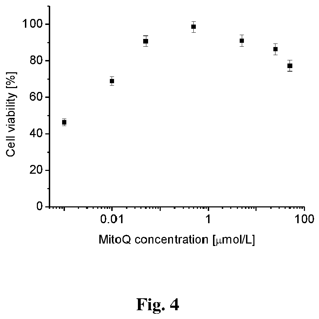

However, usage of MTA - namely the agent MitoQ (see Figure 2) - may be associated with the adverse effects, which were recently described [K. Valachová, D. Topol'ská, L. Šoltés. Protective effect of mitochondrially targeted antioxidant MitoQ on oxidatively stressed fibroblasts. Chemical Papers 72 (2018) 1223-1230]. As can be seen in Figure 4, application of MitoQ at a higher dose (concentrations 0.5 - 50 µmοl/L) resulted in a significant decrease of VH10 human-fibroblast-cell viability from 98.5 to 77.2 %.

This points to the fact that the use of the mitochondrially targeted antioxidant at a concentration higher than optimal dose could be counterproductive. Similar observations were already published [A. K. Doughan, S. I. Dikalov, Mitochondrial redox cycling of mitoquinone leads to superoxide production and cellular apoptosis. Antioxidants & Redox Signaling 9 (2007) 1825-1836; V. P. Skulachev et al., An attempt to prevent senescence: a mitochondrial approach. Biochimica et Biophysica Acta 1787 (2009) 437-461; B. D. Fink et al., Bioenergetic effects of mitochondrial-targeted coenzyme Q analogs in endothelial cells. Journal of Pharmacology and Experimental Therapeutics 342 (2012) 709-719].

### Specification of terms

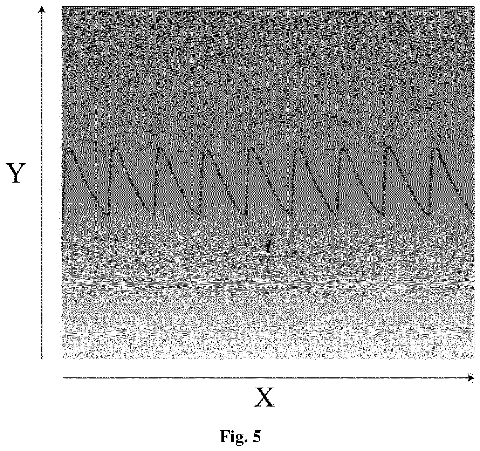

**Sustain-,** or **smart-release** of a drug or of a medicinal substance can be usually achieved by an application of a solution of these substances using a pump, which gradually administers the solution directly into the patient's vein during a certain time period. If the drug or the medicinal substance is incorporated in a carrier, which in our invention is a composite membrane, it is necessary to replace the membrane with a new one, e.g. every day. The initial flow of the drug or of the medicinal substance as well as the final flow, i.e. 24 h after application of the composite membrane, must correspond to the drug or the medicinal substance uptake into the site of (injured) skin in a dose, which provides that the concentration of the drug or the medicinal substance in the site of skin/tissue will correspond to its effective concentration - see Figure 5.

**Cytoprotection** is a process, by which a chemical compound provides protection to cells against harmful agents.

**Targeting** some parts of the patient body usually means an increase of the medication concentration by a smart-treatment-mode solely the diseased tissue. Targeted drug delivery systems have generally been emploid to optimize the regeneration of a body-tissue.

**Composite membrane** within our invention means an enclosing/separating artificially constructed membrane that provides mechanical protection for a tissue - in our case for skin - as well as renders protection against adverse noxious agents. The membrane carries more than one high- and/or low-molar-mass compounds, which serve stimulatively in treatment of chronic wounds.

**Chronic wounds** include, but are not limited to, venous stasis ulcers, arterial ulcers, diabetic ulcers, pressure ulcers, traumatic ulcers, post-surgical ulcers, small vascular wounds, wounds with bacterial biofilm, and allergy-induced wounds. Chronic wounds occur in diseases and conditions such as, but are not limited to, diabetes, vasculitis, ischemia, immune suppression, pyoderma gangrenosum, fibrosis, edema, sickle cell anemia, and peripheral arterial disease atherosclerosis.

**Wound healing** is a dynamic process involving many factors and cell types including soluble mediators, blood cells, fibroblasts, endothelial cells, and extracellular matrix. Wound healing is usually divided into several sequential phases that overlap in space and time - such as homeostasis, inflammation, granulation, tissue formation, and tissue remodeling. Chronic wounds develop as a result of defective regulation of one or more of the complex molecular and biological events involved in proper wound healing. Administration of active healing agent(s) can last for at least 21 days once a wound becomes chronic, or until the wound is visibly improved and on the way to healing.

### Background of the invention

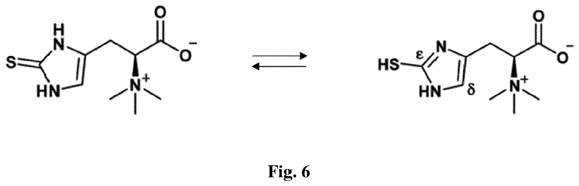

The proposal of solving the problem in the invention - "smart-released cytoprotectant targeting the inflamed tissue" - evolves from theoretical knowledge as well as from longterm practical experience of the authors of the invention:
**The cytoprotectant L-(+)-ergothioneine** (see Figure 6) is a sulfur-containing derivative of the amino acid histidine. L-(+)-Ergothioneine, synonymum (S)-α-carboxy-*N,N,N*-trimethyl-2-mercapto-1H-imidazole-4-ethanaminium, C₉H₁₅N₃O₂S, molar mass 229.3 g/mol is a non-toxic bioactive molecule. In accord with Paul and Snyder [B.D. Paul, S. H. Snyder, The unusual amino acid L-ergothioneine is a physiologic cytoprotectant. Cell Death & Differentiation 17 (2010) 1134-1140] one could say that although L-(+)-ergothioneine was isolated century ago, its physiologic function has not been so far clearly established. Because of its dietary origin, L-(+)-ergothioneine represents a vitamin, whose physiologic roles include cytoprotection. The only organisms known to synthesize L-(+)-ergothioneine are bacteria belonging to the genus Actinomycetales (example mycobacteria) and non-yeast like fungi which include members of the division Basidiomycota and Ascomycota. These microbes synthesize L-(+)-ergothioneine from L-histidine *via* an intermediate hercynine - a betaine of histidine. A sulfur group is added to hercynine to form L-(+)-ergothioneine.

Mammals acquire L-(+)-ergothioneine solely through their diet: foods such as mushrooms, black beans, red meat and oats are rich in L-(+)-ergothioneine. L-(+)-Ergothioneine is cumulated in cells and tissues frequently exposed to oxidative stress with highest levels in the millimolar range occurring in blood, crystalline lenses, liver, bone marrow and seminal fluid. Patients with rheumatoid arthritis accumulate L-(+)-ergothioneine in their synoviocytes.

L-(+)-Ergothioneine is tautomeric and in neutral aqueous solutions exists predominantly in the thione form (see Figure 6), which may account for L-(+)-ergothioneine's resistance to autoxidation. L-(+)-Ergothioneine's sulfhydryl group - in the thiol form of L-(+)-ergothioneine - underpins its antioxidant properties: L-(+)-Ergothioneine, when ingested, can scavenge reactive oxygen and nitrogen species and protect cells from a variety of apoptotic insults. L-(+)-Ergothioneine inhibits tumor necrosis factor-a induced release of the inflammatory cytokine interleukin-8 in alveolar macrophages. L-(+)-Ergothioneine taken up from the diet is retained preferentially in cells exposed to oxidative stress and involved in inflammatory responses. The presence of a high affinity transporter - OCTN1 - of L-(+)-ergothioneine in conjunction with its non-random distribution in the body strongly implies physiologic role of L-(+)-ergothioneine.

L-(+)-Ergothioneine scavenges hydroxyl radicals as well as directly absorbs UV radiation. L-(+)-Ergothioneine has an absorption spectrum in the UV range similar to DNA with a molar extinction coefficient of 1.4 × 10⁻⁴ mol⁻¹.cm⁻¹, λₘₐₓ = 257 nm, suggesting that L-(+)-ergothioneine can act as a physiological UV filter. *In vitro* and cell culture studies have identified L-(+)-ergothioneine also as a scavenger of superoxide anion radicals.

L-(+)-Ergothioneine tissue levels are maintained by its transporter - OCTN1. In the bovine lens, L-(+)-ergothioneine concentrations about 7 mmol/L, 10-times exceed those of glutathione, generally regarded as the most abundant endogenous antioxidant. In the bovine cornea, L-(+)-ergothioneine concentrations are 14-fold higher than those of glutathione, suggesting that it is the principal antioxidant in this tissue. L-(+)-Ergothioneine may provide more stable mode of cytoprotection and since it is not metabolized to any notable extent in mammalian tissues, the half-life of dietary L-(+)-ergothioneine is approximately 1 month. These properties suggest a role for L-(+)-ergothioneine as a bulwark, an ultimate defense for cells against oxidative damage. Thus, L-(+)-ergothioneine appears to be an important physiologic cytoprotectant which probably merits its designation as vitamin.

Next, the reason to use membrane compositions based on the two self-associating biopolymers - chitosan and high-molar-mass hyaluronan (HA) - could be advocated as follows.

**Chitosan** has several biochemical properties that boost wound healing including biocompatibility and non-toxicity to mammalian cells, antimicrobial activity, haemostatic activity, and anti-inflammatory activity. *N*-acetylglucosamine (NAG) units present in chitosan are dominant constituents of dermal tissue that are crucial for repairing scar tissues in addition to their contribution to the anti-inflammatory properties of chitosan. Furthermore, NAG promotes fibroblast proliferation and collagen synthesis. Chitosan plays an important role in thrombosis and blood coagulation in addition to stimulation of the cytokines and growth factors expression that stimulate angiogenesis, which is crucial to accelerate tissue restoration. Moreover, antimicrobial activity of chitosan could suppress microbial infection to wound and inhibit further complications that hamper the wound healing.

**Hyaluronan** (HA), a high-molar-mass biopolymer, is composed of repeating disaccharide units of NAG and D-glucuronic acid, connected exclusively by β-linkages. Hyaluronan is the major component of epidermis and dermis of the skin. HA is broadly used as an excellent moisturizer in cosmetic dermatology and skin-care products. In the wound healing, HA renders temporary support for assisting the transportation of nutrients during increased migration and proliferation of keratinocyte cells. Additionally, HA regulates the expression of fibroblast genes for remodeling step and dominates the angiogenesis.

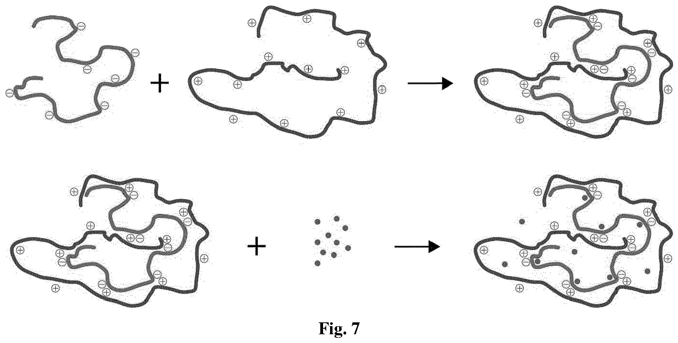

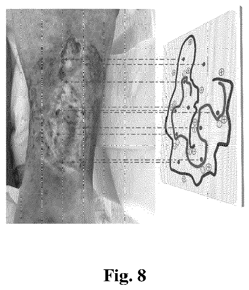

While chitosan itself forms perfect film at drying of its slightly acidic solutions (usually in 2% aqueous acetic acid), HA lacks such film forming propensities. Thus, on combining the two solutions in appropriate ratios, one may obtain a viscous solution, which on drying easily forms a perfect thin film with much higher tear resistance than a film formed only from the chitosan solution. However, if we add to the pre-formulated viscous solution of the two polymers another component (Figure 7) - in case of constructing the membrane containing cytoprotectant L-(+)-ergothioneine, the resulting product is **the composite membrane,** which is ready-to-use for treatment of difficult-to-heal chronic skin/tissue wounds. Figure 8 schematically illustrates the situation, which is self explanatory. Thus, incorporation of the molecules of the cytoprotectant L-(+)-ergothioneine into a two-component viscous solution composed of self-associating chitosan and HA results after drying in a formation of composite membranes ready-to-use to heal chronic wounds. In order to provide a desired shape and to strengthen the membrane, it is advantageous to impregnate a sparsely woven fabric/gauze or even the whole bandage with the three-component viscous solution. The cytoprotectant L-(+)-ergothioneine can be gradually released from such membranes during the extended period of time. Since after certain time L-(+)-ergothioneine from the membrane penetrates and is incorporated into the wound tissue, the used membrane can be readily removed and substituted with a new one. One of the especially important advantages of the suggested formulation is that the composite membrane containing the cytoprotectant L-(+)-ergothioneine can be preserved for unlimited time, because L-(+)-ergothioneine is not oxidized spontaneously. It is therefore possible to store sterilized membranes in a mildly wet state in an appropriate container and to apply them to a wound at any required time.

To summarize, why and how to exploit L-(+)-ergothioneine as a smart-released cytoprotectant for treatment of difficult-to-heal chronic wounds of skin/tissues we should assert state that L-(+)-ergothioneine:
**1.** bears in its molecule a positive charge, i.e. is a cation,
**2.** its molecules (at least their thiol fraction) are chemically reactive,
**3.** is transported directly into the site of inflammation or to a vicinity of stressed cells by the high affinity OCTN1 vehicle.

Low molar mass of L-(+)-ergothioneine, i.e. less than 230 g/mol, which is substantially lower than molar masses of any MTA mentioned in Figure 2, perfectly fulfils a request for a limit of molar mass of drugs circulating in the organism, i.e. up to 400 g/mol. The requirement **1,** i.e. molecule as a cation, is fulfilled by the presence of the atom N⁺ in L-(+)-ergothioneine (see Figure 6). Concerning the requirement **2,** as evident from Figure 6, molecule of L-(+)-ergothioneine is a tautomer between thione and thiol forms. While thione is a stable unoxidizable (reserve) fraction, "thiol" is an active principle, which due to the functional group -SH is an effective donor of H^{•} radical. And just H^{•} radical is a unique sufficiently reactive radical, which is able to scavenge the most reactive ROS, namely the ^{•}OH radical. Very interesting is requirement **3,** i.e. targeting the stressed cells. As can be seen in Figure 2, none of these synthetic MTA molecules fulfill and either can fulfill the requirement of preferential transfer into the site of inflammation. To address this requirement, it is necessary to have *a priori* an endogenic vehicle in the organism, which could anchor and transfer the molecule to the site of inflammation or in the vicinity of stressed cells. And exclusively for L-(+)-ergothioneine molecules the existence of such a vehicle was affirmed: it is OCTN1 [D. Gründemann et al., Discovery of the ergothioneine transporter. Proceedings of the National Academy of Sciences of the United States of America 102 (2005) 5256-5261]. In vertebrates, OCTN1 synthesis is even encoded in the DNA molecule. Moreover, only L-(+)-ergothioneine administered as a drug is transferred by OCTN1 to stressed cells [B.D. Paul, S. H. Snyder, The unusual amino acid L-ergothioneine is a physiologic cytoprotectant. Cell Death & Differentiation 17 (2010) 1134-1140].

### Summary of the invention

The invention concerns self-associating biopolymers comprising polyanionic (high-molar-mass hyaluronan) and polycationic biopolymer (chitosan), which together form a carrier for the cytoprotectant - L-(+)-ergothioneine - loosely incorporated into the carrier.

The carrier, composed of hyaluronic acid and chitosan in a ratio that ensures that the carrier has a little surplus of negative charge, loosely binds L-(+)-ergothioneine since its polarity is positive, i.e. opposite to the polarity of the carrier.

When the composite membrane is applied to the damaged tissue, L-(+)-ergothioneine loosely incorporated in the membrane gradually releases from the membrane into the site of action dependent on the L-(+)-ergothioneine concentration gradient between the membrane surface and the site of L-(+)-ergothioneine action and dependent on mutual electric attraction between the membrane and L-(+)-ergothioneine.

Based on the invention, composite membranes are used for treatment of superficial chronic tissue damages, i.e. for the treatment of difficult-to-heal wounds. Further, the invention is related to the usage of composite membranes as a material for covering wounds caused by chronic damage of the tissue on the surface of a live organism, especially skin.

Effective agents with a positive charge, which may be incorporated in the carrier, involve preferably L-(+)-ergothioneine.

To select a polyanion-type polymer for a composite membrane, high-molar-mass hyaluronic acid is especially appropriate. As the second component with a positive charge in composite membranes, it is especially appropriate to use chitosan. The advantage of using these two positively and negatively charged biopolymers for construction of membranes is the fact that opposite electric charges of biopolymers result in the enhanced mechanical stability of membranes.

According to the invention, hyaluronic acid used for the membrane construction is of a molar mass 1 × 10⁶ g/mol or higher. According to the invention, chitosan involved in the membranes is of a molar mass 5 × 10⁴ g/mol or higher, whereas the weight ratio of hyaluronic acid to chitosan in the membrane is from 1:20 to 1:40 and the amount of L-(+)-ergothioneine incorporated in the composite membrane is from 0.1 to 10 µg/cm².

Based on the invention, composite membranes can be used for treatment of chronic inflammatory skin damages and difficult-to-heal wounds as specified above. The site on the surface of the damaged tissue is covered by the membrane according to the invention. L-(+)-Ergothioneine, i.e. the agent incorporated in the composite membrane gradually diffuses into the damaged tissue, whereas the speed of the release of L-(+)-ergothioneine is dependent on L-(+)-ergothioneine concentration gradient between the membrane surface and the site of the agent's action and on the mutual electric attraction of the membrane and L-(+)-ergothioneine. When required, at the appropriate time intervals, the composite membrane is replaced with a new one.

According to the invention, composite membranes have preferred thickness up to 1 mm and are transparent to check visually on the healing process of the damaged tissue and wounds. In case of larger wounds needed to be covered with the membrane, it is appropriate to use a "scaffold", e.g. a thin-woven fabric/gauze or even all bandage, which will be impregnated from both sides with the above mentioned three-component viscous solution.

### List of Figures

**Figure 1****.** A standard 2-week treatment of the patient was not effective. As evident, after revascularization, there are still severe ulcers of the lower limb (personal communication with Priv.Doz. Michael Gorlitzer, Gesundheitszentrum Althietzing, Kupelwiesergasse 15/5, 1130 Wien, Austria).
**Figure 2****.** Some mitochondrially-targeted antioxidants: A - MitoQ, B - MitoPeroxidase, C - MitoE₂, D - MitoSOD, E - MitoTEMPOL, F - SkQ1H₂, G - SkQR1H₂, H - MitoQH₂, I - SkQ3H₂, J - SkQ4MH₂, K - SkQ5H₂ [adapted from J. Gruber, S. Fong, C.-B. Chen, S. Yoong, G. Pastorin, S. Schaffer, I. Cheah, B. Halliwell, Mitochondria-targeted antioxidants and metabolic modulators as pharmacological interventions to slow ageing. Biotechnology Advances 31 (2013) 563-592].
**Figure 3****.** Result of a 5-day treatment of the patient by applying MitoQ. A partial coverage of the wound and its granulation indicates significant progress in the treatment of ulcers of the lower limb.
**Figure 4****.** Semilogarithmic plot of the effect of MitoQ on survival of VH10 cells.
**Figure 5****.** Concentration of a drug or of a medicinal substance (blue) (axis Y) during the period of treatment (axis X) must be in a range of effective treatment level of a drug or of a medicinal substance (area marked in green), whereas it must never be in the range of levels of a drug or of a medicinal substance, which is toxic (area marked in red) and should not be in the range of therapeutically ineffective level of a drug or of a medicinal substance (area marked in yellow). The time interval between two consecutive applications of composite membranes is indicated - ***i**.*
**Figure 6****.** Thione (left) and thiol (right) forms of L-(+)-ergothioneine.
**Figure 7****.** Upper panel: The two self-associating biopolymers, namely a polyanionic high-molar-mass hyaluronan (violet) and a polycationic chitosan (green) yield a complex coacervate/polyionic complex hydrogel. Lower panel: The co-application of L-(+)-ergothioneine (blue dots) in the formation of a three-component viscous solution, i.e. the material used to construct a composite membrane.
**Figure 8****.** Composite membrane consists of a scaffold impregnated with self-associated macromolecules of chitosan (green) and high-molar-mass hyaluronan (violet) with a molecularly dispersed cytoprotectant L-(+)-ergothioneine (blue). Arrows schematically illustrate spontaneous release of L-(+)-ergothioneine molecules followed by their incorporation into the chronic wound.

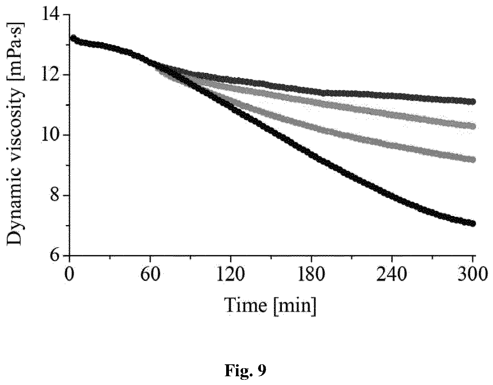

**Figure 9****.** Time-dependent changes in dynamic viscosity of hyaluronan solutions exposed to oxidative degradation by Cu(II) ions (1 µmol/L) and ascorbate (100 µmol/ L) (black). Effect of L-ergothioneine at concentrations 10 (grey), 50 (light blue), and 100 µmol/L (blue) on oxidative hyaluronan degradation. L-Ergothioneine was added to the hyaluronan reaction mixture 1 h upon the reaction onset.

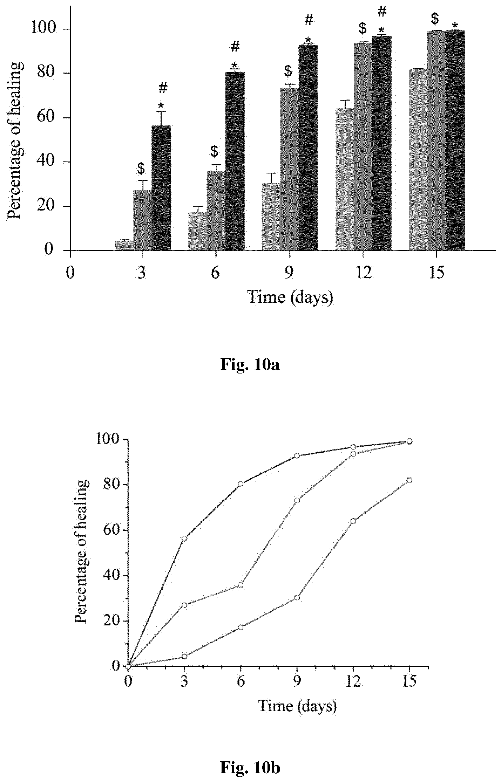

**Figure 10a****.** The areas corresponding to untreated wound (control; grey bars), the wound treated with chitosan + hyaluronan membranes (brown bars), and the wound treated with chitosan + hyaluronan + L-(+)-ergothioneine composite membranes (blue bars). The asterisk (*) indicates significant difference at p ≤ 0.05 between the control experiment and wound treated with chitosan + hyaluronan + L-(+)-ergothioneine composite membranes. The hash (#) indicates significant difference at p ≤ 0.05 between the treatments with two different membrane types. The dollar ($) indicates significant difference at p ≤ 0.05 between the control experiment and the wound treated with chitosan + hyaluronan membranes.
**Figure 10b****.** The areas corresponding to untreated wound (control; grey), the wound treated with chitosan + hyaluronan membranes (brown) and chitosan + hyaluronan + L-(+)-ergothioneine composite membranes (blue).

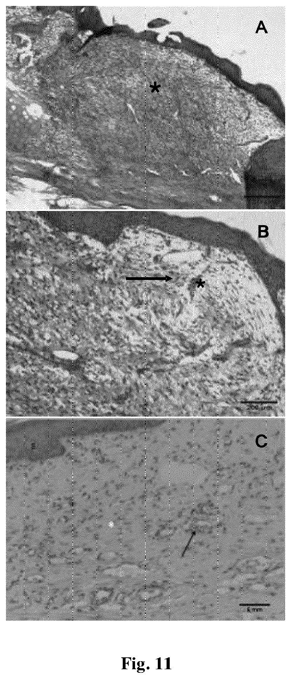

**Figure 11****.** Histograms of the preparations from (A) rabbit ear tissue from control experiment: star - vascular granular tissue; (B) rabbit ear tissue treated with chitosan + hyaluronan membranes: arrow - a site of activated fibroblasts with formation of collagen fibers, star - newly-formed veins; (C) rabbit ear tissue treated with chitosan + hyaluronan + L-(+)-ergothioneine composite membranes: E - epidermis, star - maturated granular tissue, arrow - vein.

### Embodiments of the invention

The following examples of the invention serve only for illustration and by no way limit the extent of the protection.

### Preface to example 1

Although hyaluronan/hyaluronic acid (HA) is almost omnipresent, in the human body and in other vertebrates, the highest amounts of HA are found in the extracellular matrix of soft connective tissues [G. Kogan, L. Šoltés, R. Stern, P. Gemeiner, Hyaluronic acid: a natural biopolymer with a broad range of biomedical and industrial applications. Biotechnology Letters 29 (2007) 17-25]. Almost half of the human body's high-molar-mass hyaluronan occurs in skin where, among other functions, HA immobilizes water. Skin, the largest organ of the human body, constitutes the primary protective barrier between the underlying tissues and the hostile action of the environment and/or against adverse noxious agents generated e.g. by pathogens. Below we examine protective effects of L-(+)-ergothioneine on degradation of high-molar-mass (see Example 1) in modeling chronic skin inflammation using Weissberger's biogenic oxidative system (WBOS, Scheme 1).

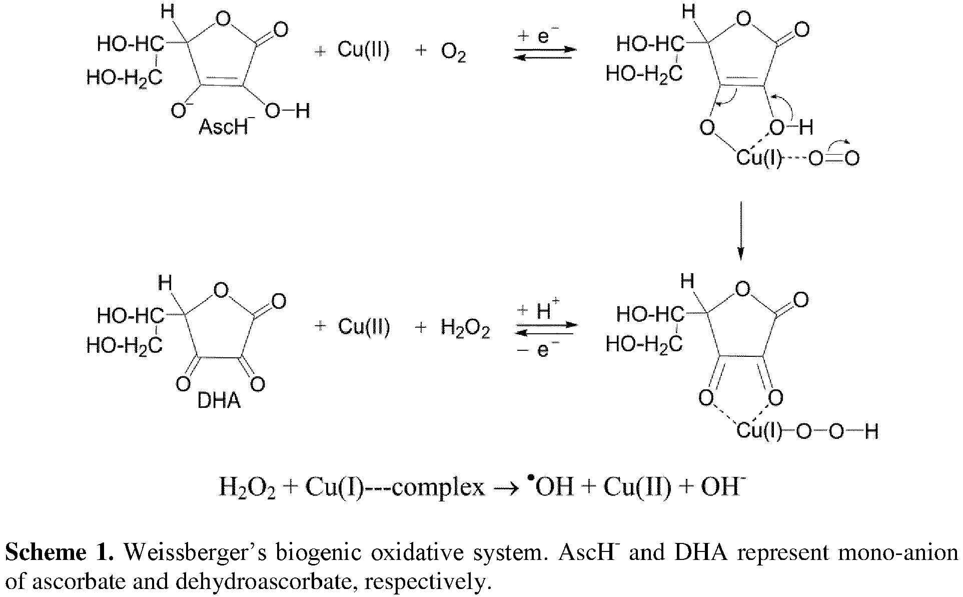

It is well established that WBOS readily generates hydroxyl radicals - one of the most reactive species - which are responsible for initiation and the subsequent perpetual propagation of free-radical oxidative degradation of high-molar-mass HA, see below: 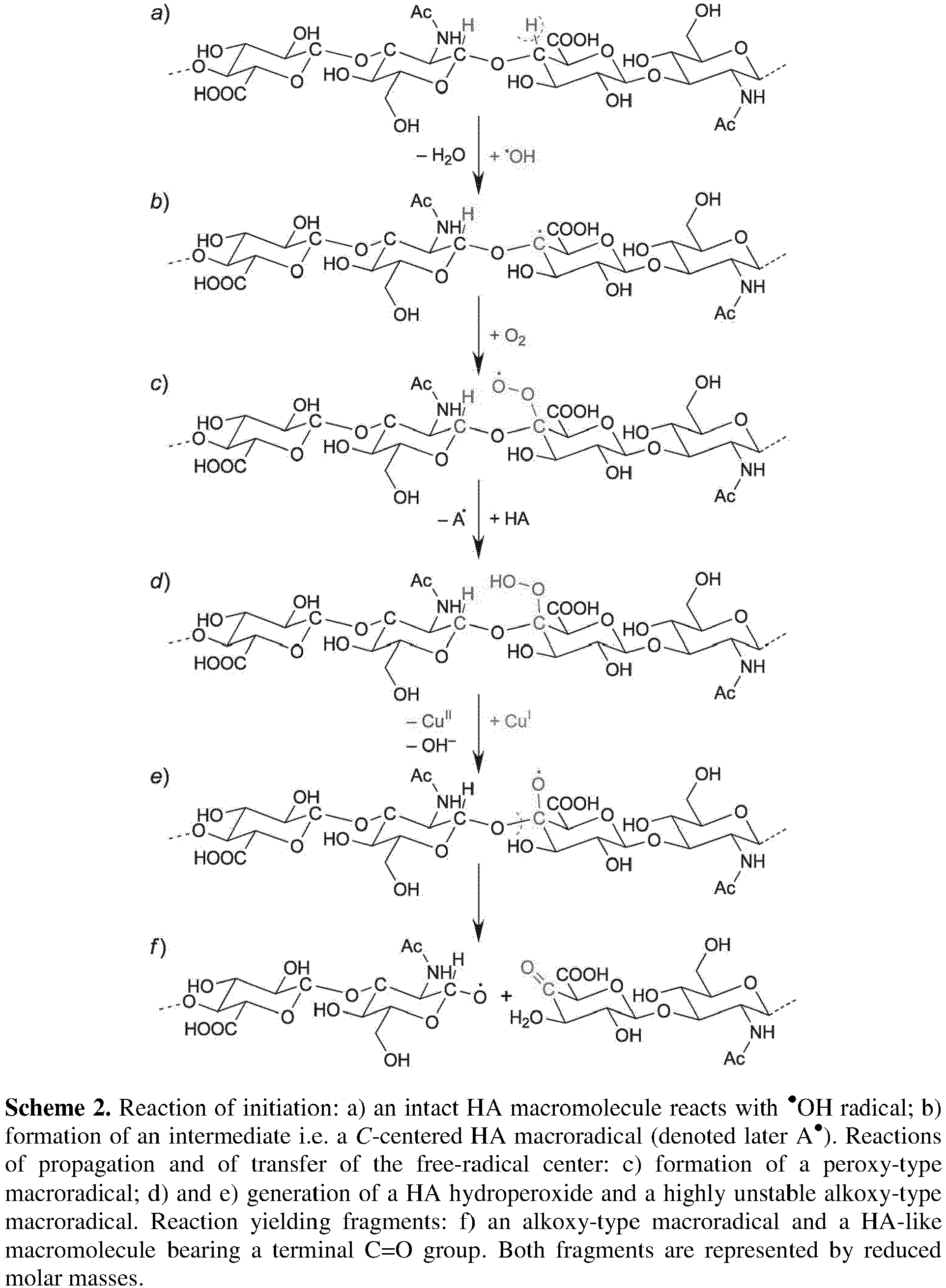 According to the reaction cascade presented in Scheme 2, the propagation steps of free-radical degradation of high-molar-mass HA can continue since both the formed peroxy- and alkoxy-type macroradicals react with native HA yielding a non ceased flux of *C*-centered HA macroradicals (i.e. A^{•}s). The fragmentation of high-molar-mass HAs, which are immunosuppressive, may lead to formation of the intermediate-sized HA-type polymers comprising 25-50 disaccharides, which however are highly immunostimulatory [G. Kogan, L. Šoltés, R. Stern, P. Gemeiner, Hyaluronic acid: a natural biopolymer with a broad range of biomedical and industrial applications. Biotechnology Letters 29 (2007) 17-25]. Thus, the discontinuation of the free-radical oxidative degradation reaction at their propagation steps by quenching the peroxy- and alkoxy-type macroradicals seems to be a logical effort.

As already demonstrated [L. Šoltés, M. Stankovská, V. Brezová, J. Schiller, J. Arnhold, G. Kogan, P. Gemeiner, Hyaluronan degradation by copper(II) chloride and ascorbate: rotational viscometric, EPR spin-trapping, and MALDI-TOF mass spectrometric investigations. Carbohydrate Research 341 (2006) 2826-2834], under properly set experimental conditions, the system consisting of high-molar-mass HA macromolecules [dissolved in aqueous NaCl (0.9 g/L)] that is involved in the WBOS initiated free-radical degradation, does not show presence of ^{•}OH radicals at time 1 h and later upon the reaction onset. Thus, after 1 h from the reaction onset, the degradation reaction should proceed exclusively by action of peroxy- and alkoxy-type (HA) macroradicals. And currently these macroradicals are effectively quenched by L-ergothioneine added into the system 1 h upon the reaction onset. The above conclusion is clearly documented in example 1.

### Example 1

### Quenching of the peroxy- and alkoxy-type (HA) macroradicals by L-ergothioneine.

The experiment was performed as follows:
**Preparation of stock and working solutions:** The HA samples (16 mg) were dissolved in 0.15 mol/L aqueous NaCl solution for 24 h in the dark in two steps: first, 4.0 mL of 0.15 mol/L NaCl was added to HA to swell and after 6 h, 3.9 or 3.85 mL of 0.15 mol/L NaCl was added. Solutions of ascorbic acid (16 mmol/L), cupric chloride (160 µmol/L) and L-(+)-ergothioneine (16, 8 and 1.6 mmol/L) were made in 0.15 mol/L aqueous NaCl.

**Uninhibited hyaluronan degradation:** First, HA degradation was induced by the oxidative system comprising CuCl₂ (1 µmol/L) with ascorbic acid (100 µmol/L). The procedure was as follows: a volume of 50 µL of CuCl₂ solution was added to the HA solution (7.90 mL), and the mixture after 30-s stirring was left to stand for 7.5 min at room temperature. Then, 50 µL of ascorbic acid solution were added to the HA mixture, which was stirred again for 30 s. The HA mixture was then immediately transferred into the viscometer Teflon^{®}cup reservoir.

**The procedure to investigate L-(+)-ergothioneine inhibitory effectiveness:** The solution of CuCl₂ (50 µL) was added to the HA solution (7.85 mL) and stirred for 30 s. The reaction mixture was left to stand for 7.5 min at room temperature, then 50 µL of ascorbic acid was added, and the mixture was stirred for 1 h. Then, 50 µL of L-(+)-ergothioneine was added to the reaction mixture and stirred again for 30 s. The resulting reaction mixture (8 mL) was transferred into the Teflon^{®} cup reservoir.

The measurements were carried out using a Brookfield LVDV-II+PRO digital rotational viscometer (Brookfield Engineering Labs., Inc., Middleboro, MA, USA), and changes in dynamic viscosity of the HA solutions were recorded at 25.0 ± 0.1 °C in 3 min intervals for 4 h. The viscometer Teflon^{®} spindle rotated at 180 rpm, i.e. at a shear rate of 237.6 s⁻¹.

The results in Figure 9 show that HA, exposed to the oxidative degradation by 1 µmol/L cupric ions and 100 µmol/L ascorbate (WBOS), reported a significant degradation (black curve). The addition of L-(+)-ergothioneine (10, 50 and 100 µmol/L) to the HA reaction mixture 1 h later, were particularly peroxy- and alkoxy-type HA macroradicals are produced, resulted in a dose-dependent retardation of HA degradation. It points to the ability of L-(+)-ergothioneine to be an effective scavenger of the above mentioned radicals.

### Example 2

### Preparation and application of composite membrane containing L-(+)-ergothioneine.

Chitosan (500 mg) of molar mass 5 × 10⁴ g/mol was dissolved in 20 mL of 2 % acetic acid (solution A). Hyaluronan (50 mg) of molar mass 1.5 × 10⁶ g/mol was dissolved in 5 mL of deionized water (solution B). Glycerol (1 mL) as a plasticizer was added to the solution A under constant stirring. L-(+)-Ergothioneine (1.47 mg) was added to the solution B. Then, the solution A was admixed into the intensively stirred solution B. Aliquot amount of the final viscous solution was: in case a) poured on a Petri dish, and the components were left to coagulate and dry at room temperature during 2 days; in case b) a scaffold - a thin-woven fabric/gauze or even the whole bandage - was impregnated with the final viscous solution. After complete drying, the membrane prepared [case a) or b)] was applied on chronic skin wounds.

### Example 3

### Healing of ischemic skin wounds.

Ischemic wounds on rabbits' ears were performed according to DiPietro's method [DiPietro, L.A.; Burns, A.L. Wound Healing Methods and Protocols; Humana Press: New York, NY, USA, 2003]. Inside of each rabbit's ear, two lacerations with a size of ca. 1 cm × 1 cm and a complete removal of skin tissue were performed. Rabbits were divided into three groups of three animals: first group - control (wound was covered with bandage); second group - animals treated with chitosan + hyaluronan membrane; and third group - animals treated with chitosan + hyaluronan + L-(+)-ergothioneine composite membrane.

The progress in healing the skin wounds during the 15 day-experiment is demonstrated in Figures 10a and 10b. The wounds were objectively assessed on the days 0, 3, 6, 9, 12, and 15 day of the experiment by measuring the area of wound.

Along with an objective assessment of wound healing by measuring the wound area at the fixed days of experiment (3, 6, 9, 12, and 15), replacement of the membranes with the fresh ones was performed on days 3, 6, 9, and 12. Thus, the period of application the new membranes lasted three days.

A subjective, visual evaluation of the healing process indicated a broader re-epithelization of skin wounds already on the 6^{th} day when the wound was covered with whichever used membrane compared to the control experiment. The continued healing process inspection on day 9 exhibited significant progress especially when the wound healing was carried out using the chitosan + hyaluronan + L-(+)-ergothioneine composite membranes. On the 12^{th} day, subjectively, the wounds treated by using any type of membrane compared to untreated wounds could have been looked upon as fully recovered skin.

After finishing the experiments, the reformed skin tissues underwent a complex histological evaluation: the preparations isolated were, in accordance with the standard protocol, stained with hematoxylin and eosine.

The wound in Figure 11, panel A, corresponds to inflammatory phase of healing. The wound is composed of hypercellular nonspecific granular tissue with prevalence of fibroblasts and amorphic collagen; Figure 11, panel B, illustrates inflammatory/proliferative phase of wound healing. The wound comprises myofibroblasts, nonspecific granular tissue with loss of cellularity [amorphic collagen, see panel B, was replaced with the fibrous one]. Figure 11, panel C, displays the remodelation phase of wound healing. The wound is composed of hypocellular nonspecific granular tissue with prevalence of myofibroblasts and fibrous collagen.

## Claims

1. A composite membrane for a sustain-/smart-released cytoprotectant in treating chronic tissue damages, composed of two self-associating biopolymers with at least one cytoprotectant incorporated therein.

2. The composite membrane of claim 1, **characterized in that** the biopolymers are high-molar-mass hyaluronic acid and chitosan.

3. The composite membrane of claim 1, **characterized in that** the cytoprotectant is L-(+)-ergothioneine.

4. The composite membrane of claim 3, **characterized in that** L-(+)-ergothioneine is an integrated component of the composite membrane.

5. The composite membrane of claim 2, **characterized in that** the hyaluronic acid is of molar mass 1 × 10⁶ g/mol or higher and chitosan is of molar mass 5 × 10⁴ g/mol or higher.

6. The composite membrane of claim 5, **characterized in that** the weight ratio of hyaluronic acid to chitosan in the composite membrane is from 1:20 to 1:40.

7. The composite membrane of claims 3 and 6, **characterized in that** the L-(+)-ergothioneine amount incorporated in 1 cm² of the composite membrane is from 0.1 to 10 µg.

8. The composite membrane of claim 7, **characterized in that** the ready-for-use composite membrane, when applied for treatment of chronic skin damages and difficult-to-heal wounds, gradually releases L-(+)-ergothioneine in accord to the gradient of L-(+)-ergothioneine concentration between the surface of the membrane and the site of its action and on mutual electric attraction between the membrane and L-(+)-ergothioneine.

9. The composite membrane of claim 7, **characterized in that** its characteristic thickness is up to 1 mm and is transparent to check visually on the healing process of the damaged tissue and wounds.

10. The composite membrane of claim 7, **characterized in that**, in case of larger surface of wounds, the membrane is constructed by using a scaffold such as a thin-woven fabric/gauze or even the whole bandage, which is impregnated from both sides with the components according to claim 4: L-(+)-ergothioneine, high-molar-mass hyaluronic acid and chitosan.

11. The composite membrane of claim 1, for use in treatment of chronic inflammatory tissue damage on the surface of the organism, which is caused by diseases selected from diabetes, vasculitis, ischemia, immune suppression, pyoderma gangrenosum, fibrosis, edema, sickle cell anemia, and peripheral arterial disease atherosclerosis, and chronic wounds are selected from venous stasis ulcers, arterial ulcers, diabetic ulcers, pressure ulcers, traumatic ulcers, post-surgical ulcers, small vascular wounds, wounds with bacterial biofilm, and allergy-induced wounds.

12. The composite membrane of claim 1, for use in treating chronic wounds, wherein the chronic wounds occur in diseases and conditions selected from diabetes, vasculitis, ischemia, immune suppression, pyoderma gangrenosum, fibrosis, edema, sickle cell anemia, and peripheral arterial disease atherosclerosis.

## Amended claims

### Amended claims in accordance with Rule 137(2) EPC.

1. A composite membrane for a sustain-/smart-released cytoprotectant in treating chronic tissue damages, composed of two self-associating biopolymers, which are high-molar-mass hyaluronic acid and chitosan, with L-(+)-ergothioneine incorporated therein.

2. The composite membrane of claim 1, **characterized in that** L-(+)-ergothioneine is an integrated component of the composite membrane.

3. The composite membrane of claim 1, **characterized in that** the hyaluronic acid is of molar mass 1 × 10⁶ g/mol or higher and chitosan is of molar mass 5 × 10⁴ g/mol or higher.

4. The composite membrane of claim 3, **characterized in that** the weight ratio of hyaluronic acid to chitosan in the composite membrane is from 1:20 to 1:40.

5. The composite membrane of claims 1 and 4, **characterized in that** the L-(+)-ergothioneine amount incorporated in 1 cm² of the composite membrane is from 0.1 to 10 µg.

6. The composite membrane of claim 5, **characterized in that** the ready-for-use composite membrane, when applied for treatment of chronic skin damages and difficult-to-heal wounds, gradually releases L-(+)-ergothioneine in accord to the gradient of L-(+)-ergothioneine concentration between the surface of the membrane and the site of its action and on mutual electric attraction between the membrane and L-(+)-ergothioneine.

7. The composite membrane of claim 5, **characterized in that** its characteristic thickness is up to 1 mm and is transparent to check visually on the healing process of the damaged tissue and wounds.

8. The composite membrane of claim 5, **characterized in that**, in case of larger surface of wounds, the membrane is constructed by using a scaffold such as a thin-woven fabric/gauze or even the whole bandage, which is impregnated from both sides with the components according to claim 4: L-(+)-ergothioneine, high-molar-mass hyaluronic acid and chitosan.

9. The composite membrane of claim 1, for use in treatment of chronic inflammatory tissue damage on the surface of the organism, which is caused by diseases selected from diabetes, vasculitis, ischemia, immune suppression, pyoderma gangrenosum, fibrosis, edema, sickle cell anemia, and peripheral arterial disease atherosclerosis, and chronic wounds are selected from venous stasis ulcers, arterial ulcers, diabetic ulcers, pressure ulcers, traumatic ulcers, post-surgical ulcers, small vascular wounds, wounds with bacterial biofilm, and allergy-induced wounds.

10. The composite membrane of claim 1, for use in treating chronic wounds, wherein the chronic wounds occur in diseases and conditions selected from diabetes, vasculitis, ischemia, immune suppression, pyoderma gangrenosum, fibrosis, edema, sickle cell anemia, and peripheral arterial disease atherosclerosis.
